(19) Europäisches Patentamt
European Patent Office
Office européen des brevets

(11) Publication number: 0 475 231 A1

# (12) EUROPEAN PATENT APPLICATION

(21) Application number: 91114730.4

(51) Int. Cl.[5]: C07D 403/04, A61K 31/55

(22) Date of filing: 02.09.91

(30) Priority: 10.09.90 US 579526

(43) Date of publication of application:
18.03.92 Bulletin 92/12

(84) Designated Contracting States:
AT BE CH DE DK FR GB IT LI LU NL SE

(71) Applicant: F. HOFFMANN-LA ROCHE AG
Postfach 3255
CH-4002 Basel(CH)

(72) Inventor: Bellemin, Anne Roberte
16 Petunia Drive, Apt. 2 K
North Brunswick, N.J. 08902(US)
Inventor: Hsu, Ming-Chu
445 East 86th Street, Apt. 15 G
New York, N.Y. 10028(US)
Inventor: Keith, Dennis Dalton
8 Mendl Terrace
Montclair, N.J. 07042(US)
Inventor: Sim, Iain Stuart
63 Beverly Road
Upper Montclair, N.J. 07043(US)
Inventor: Tam, Steve Yik-Kai
13 Evergreen Road
West Caldwell, N.J. 07006(US)

(74) Representative: Mahé, Jean et al
Postfach 3255 Grenzacherstrasse 124
CH-4002 Basel(CH)

## (54) Benzodiazepines.

(57) Benzodiazepines of formula

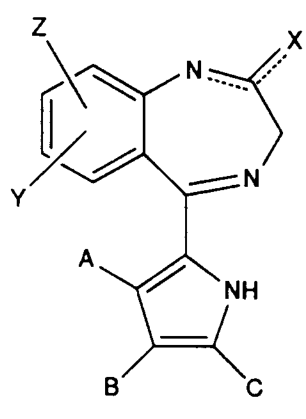

I

wherein

| | |
|---|---|
| X | is N(R,R[1]), $OR^4$ or $SR^5$, |
| R and $R^1$ | are N($R^2$,$R^3$) or substituted lower alkyl or an optionally substituted $C_3$-$C_7$-cycloalkyl, aryl, aralkyl or $C_2$-$C_6$-aliphatic hydrocarbon group, this aliphatic group having one or more double or triple bond, or one of R and $R^1$ is hydrogen, or |
| N(R,$R^1$) | is an optionally substitued heterocycle with one or more heteroatoms, |
| $R^2$ and $R^3$ | are H, lower alkyl, aryl or aralkyl, |
| $R^4$ | is lower alkyl or has one of the meanings of R or $R^1$ with the exception of H and of N($R^2$,$R^3$), |
| $R^5$ | is H or lower alkyl or has one of the meanings of R or $R^1$ with the exception of N($R^2$,$R^3$), |

EP 0 475 231 A1

Y and Z are H, $NO_2$, $N(R^2,R^3)$, halogen, $CF_3$, OH, CN or lower alkyl or alkoxy, and
A, B and C are H, $CH_3$, $C_2H_5$, F or Cl,

and pharmaceutically acceptable salts thereof are useful as medicaments especially for the treatment or prophylaxis of viral infections, particularly of retroviral infections, such as HIV 1 and/or HIV 2 infections, or for protecting cells against such infections.

The present invention relates to benzodiazepines of formula

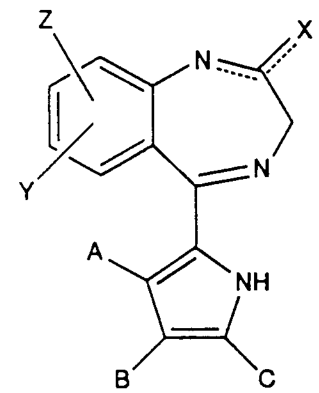

I

wherein

| | |
|---|---|
| X | is $N(R,R^1)$, $OR^4$ or $SR^5$, |
| R and $R^1$ | are $N(R^2,R^3)$ or substituted lower alkyl or an optionally substituted $C_3$-$C_7$-cycloalkyl, aryl, aralkyl or $C_2$-$C_6$-aliphatic hydrocarbon group, this aliphatic group having one or more double or triple bond, or one of R and $R^1$ is hydrogen, or |
| $N(R,R^1)$ | is an optionally substitued heterocycle with one or more heteroatoms, |
| $R^2$ and $R^3$ | are H, lower alkyl, aryl or aralkyl, |
| $R^4$ | is lower alkyl or has one of the meanings of R or $R^1$ with the exception of H and of N-$(R^2,R^3)$, |
| $R^5$ | is H or lower alkyl or has one of the meanings of R or $R^1$ with the exception of $N(R^2,R^3)$, |
| Y and Z | are H, $NO_2$, $N(R^2,R^3)$, halogen, $CF_3$, OH, CN or lower alkyl or alkoxy, and |
| A, B and C | are H, $CH_3$, $C_2H_5$, F or Cl, and pharmaceutically acceptable salts thereof. |

Objects of the present invention are the above compounds per se and for use as a therapeutically active agent, especially for the treatment or prophylaxis of viral infections, particularly of retroviral infections, such as HIV 1 and/or HIV 2 infections, or for protecting cells against such infections;

further a process for the manufacture of these compounds and medicaments containing one of such compounds and, optionally, a second antiviral agent, especially a reverse transcriptase inhibitor, such as ddC, AZT, a HIV-protease inhibitor, $\alpha$-, $\beta$- and/or $\gamma$-interferon, interleukin-2 and/or GM-CSF, and

the use of these compounds for the manufacture of medicaments especially for the treatment or prophylaxis of viral infections, particularly of retroviral infections, such as HIV 1 and/or HIV 2 infections, or for protecting cells against such infections.

As used therein, the term "lower" refers to straight or branched hydrocarbon chains with up to 7 C atoms, such as methyl, ethyl, propyl and isopropyl.

In the above general formula the dotted lines are intended to indicate the presence of single or double bonds in these positions. The relative positions of the single and double bonds is determined by whether the group N-C-X is capable of existing in tautomeric form. The tautomeric forms of the compounds of formula I are included in the scope of this invention. Moreover, when the double bond is exocyclic (N-C=X), it is understood that there is an H on the nitrogen. All the stereoisomeric forms of these compounds are also included in the scope of the invention.

Pharmaceutically acceptable salts may be those with organic acids, e.g. lactic, acetic, malic or p-toluenesulfonic acid; or salts with mineral acids, such as hydrochloric or sulfuric acid.

Examples of "aryl" groups, taken alone or in combination, e.g. in "aralkyl" are phenyl, tolyl and xylyl. Examples of "heteroatoms" in a "heterocycle" $N(R,R^1)$ are N, O and S.

Examples of substituents in lower alkyl, ccloalkyl, aryl, aralkyl and $C_2$-$C_6$-aliphatic groups R to $R^5$ are: $OR^3$, $SR^2$, halogen, $N(R^2,R^3)$, CN, $N^3$, $COOR^2$ and $COR^2$. A further example of a substituent of the aryl groups R, $R^1$, $R^4$ and $R^5$ and of the aralkyl groups $R^4$ and $R^5$ is $NO_2$.

Preferred compounds are those wherein X is $SCH_3$, SH, $OCH_3$, $NHNH_2$, NH(cyclopropyl), $NHCH_2CH(OCH_3)_2$, $NHCH_2CH_2OH$, $NHCH_2CH_2NH_2$, $NHCH_2C_6H_5$, $N(CH_3)_2$, $N(CH_2CH_3)_2$, $NHCH_2CH=CH_2$, $NHCH_2C\equiv CH$, $NHC_6H_5$, 2-hydroxymethylpyrrolidin-1-yl, 1-pyrrolidinyl, 3-hydroxypyrrolidin-1-yl, 1-piperidinyl, 1-piperazinyl or 4-morpholinyl, Y is Cl, $NO_2$, $NH_2$ or H, and Z, A, B and C are H.

Particularly preferred compounds are those wherein Y is 7-chloro; A, B, C and Z are H and X is $SCH_3$,

SH, $OCH_3$, $NHNH_2$, NH(cyclopropyl), $NHCH_2CH(OCH_3)_2$, $NHCH_2CH_2OH$, $NHCH_2CH_2NH_2$, $NHCH_2C_6H_5$, N-$(CH_3)_2$, $N(CH_2CH_3)_2$, $NHCH_2CH=CH_2$, $NHCH_2C\equiv CH$, $NHC_6H_5$, 1-pyrrolidinyl, 3-hydroxypyrrolidin-1-yl, 1-piperidinyl or 1-piperazinyl.

Even more preferred compounds are those compounds in which X is $NHNH_2$, $N(CH_3)_2$, $NHCH_2C\equiv CH$, $NHCH_2CH=CH_2$, $NHCH_2CH(OCH_3)_2$, 1-pyrrolidinyl, 3-hydroxypyrrolidinyl, 1-piperidinyl or 1-piperazinyl.

Examples of such preferred compounds are the following:

7-chloro-N,N-dimethyl-5-(1H)-pyrrol-2-yl)-3H-1,4-benzodiazepin-2-amine,
7-chloro-5-(1H-pyrrol-2-yl)-2.(1-pyrrolidinyl)-3H-1,4-benzodiazepine,
7-chloro-N-cyclopropyl-5-(1H-pyrrol-2-yl)-3H-1,4-benzodiazepin-2-amine,
7-chloro-N-(2,2-dimethoxyethyl)-5-(1H-pyrrol-2-yl)-3H-1,4-benzodiazepin-2-amine,
7-chloro-N-(2-hydroxyethyl)-5-(1H-pyrrol-2-yl)-3H-1,4-benzodiazepin-2-amine,
rac-1-[7-chloro-5-(1H-pyrrol-2-yl)-3H-1,4-benzodiazepin-2-yl]-3-pyrrolidinol,
7-chloro-N-(2-propenyl)-5-(1H-pyrrol-2-yl)-3H-1,4-benzodiazepin-2-amine,
7-chloro-N-(2-propynyl)-5-(1H-pyrrol-2-yl)-3H-1,4-benzodiazepin-2-amine,
7-chloro-2-(1-piperidinyl)-5-(1H-pyrrol-2-yl)-3H-1,4-benzodiazepine,
7-chloro-2-hydrazino-5-(1H-pyrrol-2-yl)-3H-1,4-benzodiazepine,
7-chloro-2-methoxy-5-(1H-pyrrol-2-yl)-3H-1,4-benzodiazepine.

The compounds of the invention can be prepared by

a) reacting an amide of formula II or a phosphorylated derivative thereof of formula III

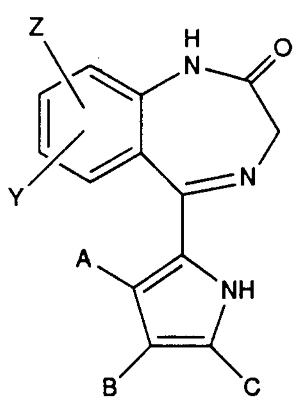

II

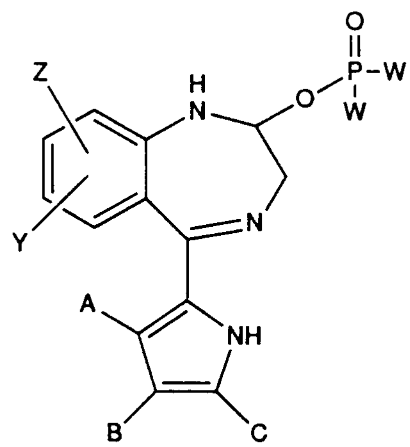

III

wherein W is lower alkoxy or an amine functional group, with an amine of formula $HN(R,R^1)$, or

b) reacting an amide II with a thiation reagent and,

c) if desired, S-alkylating a compound of formula I, wherein X is S with a compound of formula $R^5$-L wherein L is a leaving group, and

d) if desired, reacting a compound of formula I, wherein X is S-$R^5$ with an alkali metal alkoxide, and

e) if desired, reacting a compound of formula I, wherein X is S, $SR^5$ or $OR^4$ with an amine $HN(R,R^1)$, and

f) if desired, adding a substituent in the benzene moiety of a compound of formula I or interverting functional substituents in said moiety.

The reaction a) of an amide II with an amine $HN(R,R^1)$ can be carried out in an inert solvent, such as THF, toluene or dioxane, in the presence of an acid catalyst, such as p-toluenesulfonic acid or a Lewis acid, e.g. $TiCl_4$ or $SnCl_4$.

The amides II can be obtained from corresponding ketones IV which in turn are prepared by reacting an amine V with a nitrile VI, where T is a pyrrole protecting group

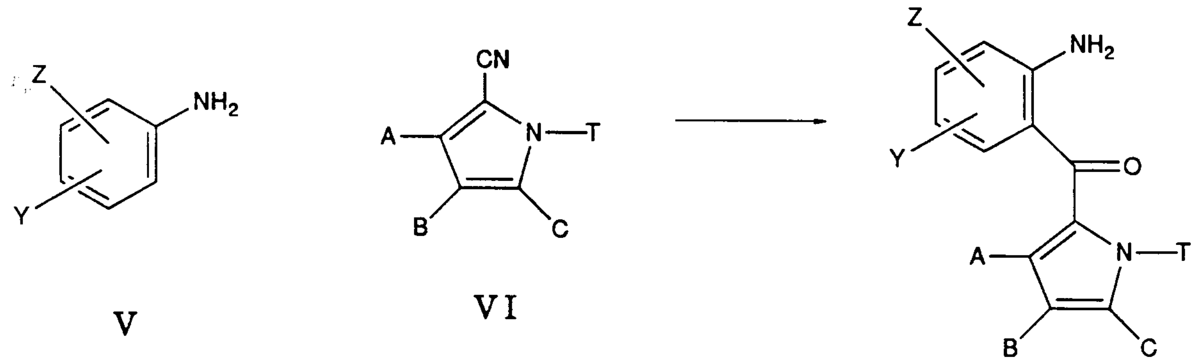

V VI IV

The intermediates III wherein W is e.g. methoxy, ethoxy, morpholino or dimethylamino are prepared by phosphorylation of the corresponding amides II. A compound III can be used in the synthesis of a compound I, e.g. wherein X is e.g. $NHNH_2$ by reaction with hydrazine.

According to b) above, a ketone II is reacted with a thiation reagent, such as $P_4S_{10}$ or 2,4-b is(4-methoxyphenyl)-1,3-dithia-2,4-diphosphetane-2,4-disulfite (Lawesson's reagent), in an inert solvent, e.g. THF, toluene, pyridine or dioxane, usually at elevated temperature, e.g. between 50 and 100 ° C or in an ultrasonic water bath.

The leaving group L in c) above is Cl, Br, J or a sulfonyloxy group, e.g. benzenesulfonyloxy or methanesulfonyloxy. The S-alkylation c) may be conducted in the presence of a base, such as an alkali metal carbonate, e.g. $Na_2CO_3$ or $NaHCO_3$, or NaH, or an organic base, e.g. pyridine or triethylamine, and in an inert solvent, e.g. dioxane, DMF, toluene, THF or 2-propane.

The reaction d) can e.g. be effected with $NaOC_2H_5$ or $LiOCH_3$ in an inert solvent, e.g. an alcohol from which the alkoxide is derived, DMF, DMSO or pyridine.

The reaction e) can be carried out in an inert solvent, e.g. THF, dioxane, DMF or pyridine.

Example of substitution reactions f), which can also be effected in the starting material or intermediate stage, are nitration, halogenation or oxidation. Examples of interconversion of functional groups on the benzene ring are that of $NO_2$ to $NH_2$, of $NH_2$ to halogen and of halogen to ether.

The compounds I and their salts have useful antiviral, especially antiretroviral activity, particularly against HIV, the virus implicated in the development of AIDS and related diseases such as ARC (AIDS related complex). These compounds also inhibit HIV replication by inhibiting such important HIV viral functions as TAT (transactivating transcriptional) activity.

The compounds I were tested for the inhibition of HIV-cytopathic effect and the reduction of cell-associated viral antigens in an experiment run as follows:

High titer virus stocks (HIV-1 NIT strain) were grown in $CD_4$ + CR10 cells in RMPI-1640 media (Gibco Laboratories) supplemented with 10% fetal calf serum and 0.1 mg/ml Gentamicin. The collected media were filtered and the virus isolates were concentrated 100 fold and stored at -80 ° C. $CD_4$ + CEM cells, propagated in the same medium, were incubated for 60 minutes at 37 ° C with diluted stock virus at multiplicity of infection (MOI) equal to 1. Cells were washed with phosphate buffered saline and resuspended in the medium at 2 x $10^5$ cells/ml. Various quantities of the test compounds in DMSO were added. Four days after infection, the number of live cells were counted by trypan blue exclusion (Proc. Natl. Acad. Sci. 83, 1986, 1911). At the same time, aliquots of cells were fixed with acetone and stained with antibodies from AIDS patients, followed by a second staining with fluorescein-conjugated goat anti-human IgG (Cappel). Cells stained with the fluorescent antibody were counted using a fluorescence microscope and the results were expressed as percentage of the total number of cells counted (J. Med. Virol. 19, 1986, 325).

The results of this anti-HIV activity test, expressed in $IC_{50}$ ($\mu$M), i.e. the concentration of the test compound which reduces the number of immuno-fluorescent positive cells by 50%, are given in Table I below.

For cytotoxicity testing, CEM cells were treated with a compound of formula I at similar concentrations and the toxicity of the compound was measured by the live-cell count.

Table I

| X | $IC_{50}$ (μM) |
|---|---|
| $SCH_3$ | 5-10 |
| $OCH_3$ | 1 |
| $NHNH_2$ | 1 |
| $NHCH_2Ph$ | 3 |
| NH(cyclopropyl) | 0.8 |
| $NHCH_2CH(OCH_3)_2$ | 1-5 |
| $NHCH_2CH_2OH$ | 2-5 |
| $N(CH_3)_2$ | 1-3 |
| 1-piperidinyl | 2 |
| 1-pyrrolidinyl | 0.8-5 |
| 3-OH-1-pyrrolidinyl | 1-5 |

The compounds presented in this Table have 50% cytotoxicity values, as measured by live cell count, greater than 10 μM. These compounds are thus selective anti-HIV agents and not general cytotoxic agent.

The compounds of formula I were also tested for anti-HIV-TAT activity in an assay comprising the following steps:

(a) putting both the expression of the Secreted Alkaline Phosphatase (SeAP) gene and the viral transactivator TAT gene under the control of the HIV promoter LTR responsive to the action of the HIV transactivator TAT;

(b) transfecting cultured mammalian cells with plasmids which contain the gene constructs of (a) above and cause cellular production of the transactivating factor TAT and SeAP;

(c) adding the agent to be tested, here the compounds of formula I; and determining the amount of SeAP produced, by measuring SeAP enzymatic activity, whereby inhibition of SeAP production correlates with the anti-TAT inhibition activity.

In this assay, the inhibition of SeAP positively correlates with anti-TAT activity. The greater the ability of an agent to inhibit SeAP, the greater is its anti-TAT activity.

Specifically, with respect to the results reported below, the anti-HIV-TAT assay was run as follows:

At 24 hours post transfection 1, 10, 25 and 50 μM of a test compound of formula I was added to the culture media of COS cells transfected with two plasmids, one containing the reporter gene which codes for SeAP under control of HIV-LTR, and the other containing the HIV-TAT gene also under control of HIV-LTR. The alkaline phosphatase activity of the media was assayed 48 hours after addition of test compound with a colorimetric assay using p-nitrophenylphosphate as the substrate. The anti-TAT activity is measured by the percent inhibition of SeAP gene expression under the control of HIV-LTR versus the percent inhibition of SeAP gene under RSV-LTR, which does not respond to TAT.

The results in Table II below show that the compounds of formula I are specific inhibitors of HIV-TAT-regulated gene expression without non-specific cytotoxic effects.

The specificity of the compounds of formula I as TAT inhibitors was demonstrated with a parallel assay in which the SeAP gene expression is put under control of the Rous sarcoma virus (RSV)-LTR which does not respond to TAT. This assay thus eliminates the possibility that the compounds of formula I are either general cytotoxic agents or inhibit the activity of SeAP.

The anti-HIV-TAT activities of the test compounds were determined by measuring the amount of alkaline phosphatase in the supernatant media of cultures of cells in which SeAP gene expression was under the control of the HIV LTR promoter. The specific inhibitory activities of the test compounds were calculated according to the formula:

$$100[(1-A/B)-(1-C/D)]$$

where A and B are the alkaline phosphatase activites produced by HIV-LTR/SeAP in the presence and absence, respectively, of test compound, and C and D are the alkaline phosphatase activities produced by RSV-LTR/SeAP in the presence and absence, respectively, of test compound. The concentrations tested ranged from 1-50 μM. The results provided are the average of at least three tests. The test compound was added 24 hours after cells were transfected with the plasmids when SeAP specific mRNA and protein were already present and the protein was very stable. Therefore, 100% inhibition would not be observed with this assay procedure.

Table II

| X | Anti-HIV-TAT activity |
|---|---|
| $SCH_3$ | Low/Medium |
| $OCH_3$ | Medium |
| $NHNH_2$ | High |
| $NHCH_2Ph$ | Low/Medium |
| NH(cyclopropyl) | Medium |
| $NHCH_2CH(OCH_3)_2$ | Medium/High |
| $NHCH_2CH_2OH$ | Medium |
| $NHCH_2CH_2NH_2$ | Medium |
| $N(CH_3)_2$ | Medium/High |
| $N(CH_2CH_3)_2$ | Low/Medium |
| 1-piperidinyl | Medium/High |
| 1-pyrrolidinyl | Medium/High |
| 3-OH-1-pyrrolidinyl | High |
| $NHCH_2CH=CH_2$ | Low |
| 1-piperazinyl | Low |

Compounds of the present invention were also examined for their ability to inhibit HIV-1 RT using recombinant DNA-derived enzyme (S.F.J. Le Grice and F. Gruninger-Leitch, European Journal of Biochemistry 187:307-314 (1990)) in 100ml volume assay mixtures. Mixtures contained 50mM Tris-HCl (pH 8.0), 25mM KCl, 4mM $MgCl_2$, 5mM dithiothreitol (DTT), 0.05% Triton X-100, 5mM $^3$H-TTP (2Ci/mmol), 1mg poly-(rA)•oligo(dT)$_{10}$ (20:1) as template/primer, test compound at the desired concentration, and an appropriate quantity of HIV RT, usually 0.01 to 0.1mg. The assay mixtures were incubated at 37° C for 10 minutes and the reactions were stopped by the addition of 10% trichloroacetic acid (TCA). Yeast RNA (100mg per reaction) was added and the reaction products were precipitated at 4° C for 30 minutes. TCA-precipitated material was collected by filtration and the bound radioactivity determined by counting using a liquid scintillation counter. The concentration of compound required to inhibit the activity of HIV-RT by fifty percent ($IC_{50}$) was determined from a multiple dose-effect analysis program (J. Chou and I. C. Chou, Dose Effect Analysis with Microcomputers, Elsevier Sciences Publishers, Amsterdam 1987). The results are shown in Table III.

Table III

| X | $IC_{50}$ ($\mu$M) |
|---|---|
| $SCH_3$ | 1.6 |
| $OCH_3$ | 1.5 |
| $NHCH_2Ph$ | 2.6 |
| NH(cyclopropyl) | 5.4 |
| $NHCH_2CH_2OH$ | 8.9 |
| $N(CH_3)_2$ | 7.3 |
| 1-piperidinyl | 10.4 |
| 1-pyrrolidinyl | 10.2 |
| 3-OH-1-pyrrolidinyl | 6.4 |
| $NHCH_2CH=CH_2$ | 4.2 |
| $NHCH_2C\equiv CH$ | 2.0 |
| NHPh | 15.8 |
| piperazinyl | 18.0 |

With respect to human patients infected with HIV, and patients with symptomatic or asymptomatic HIV infections, an antivirally-effective amount of a compound of formula I or a salt thereof is in the range of from about 0.5 to 40 mg/kg, preferably from about 2 to 15 mg/kg, more preferably from about 3 to 5 mg/kg body weight per day. In unit dosage form, for a 55 kg patient, this would be an amount of from about 27.5 to 2.200 mg per day, preferably about 170 mg per day. This dosage may be administered parenterally or

orally in one or more doses at various intervals daily, preferably orally once daily.

The compounds may also be administered with other antiviral and/or biological response modifiers. For example, the compounds of formula I may be administered with known HIV-RT inhibitors such as ddC, AZT and ddI or other inhibitors which act against other HIV proteins such as protease, integrase and RNAase, as well as with biological modifiers such as $\alpha$-, $\beta$- or $\gamma$- interferon or a combination thereof, interleukin-2 and GM-CSF. The dosages of ddC and AZT used in AIDS or ARC human patients have been published. A virustatic range of ddC is generally between 0.05 to 1.0 $\mu$M. A range of about 0.005-0.25 mg/kg body weight is virustatic in most patients. The preliminary dose ranges for oral administration are somewhat broader, for example 0.001 to 0.25 mg/kg given in one or more doses at intervals of 2, 4, 6, 8 or 12 hours. Currently 0.01 mg/kg body weight ddC given every 8 hours is preferred. When given in combined therapy, the other anti-HIV compounds may be given at the same time as a compound of formula I or the dosing may be staggered as desired. The two (or more) drugs may also be combined in a composition. Doses of each drug may be less when used in combination then when they are used as a single agent.

It is possible for the compounds of the invention to be administered alone in solution. However, it is preferred that the active ingredients be administered in a pharmaceutical formulation or composition. These formulations comprise at least one active ingredient together with one or more pharmaceutically acceptable carrier and excipient and may optionally include other therapeutic agents, for example a protease inhibitor. These carriers include those suitable for oral, rectal, nasal, topical, buccal, sublingual, vaginal or parenteral (including subcutaneous, intramuscular, intravenous and intradermal) administration.

Examples of compositions of the invention are solutions of the active ingredient(s), e.g. in water or saline; capsules, e.g. soft gelatine capsules; sachets or tablets, each containing a pre-determined amount of the active ingredient, e.g. as granules; solutions or suspensions in an aqueous liquid or in an oil-in-water emulsion or a water-in-oil liquid emulsion. Tablets may include one or more of lactose, microcrystalline cellulose, colloidal silicon dioxide, croscarmellose sodium, magnesium stearate, stearic acid and other excipients, colorants and pharmacologically compatible carriers. Formulations suitable for topical administration include lozenges comprising the active ingredient in a flavor, usually sucrose and acacia or tragacanth; pastilles comprising the active ingredient in an inert basis such as gelatin and glycerin, or sucrose and acacia; and mouthwashes comprising the active ingredient in a suitable liquid carrier. Formulations for rectal administration may be presented as a suppository with a suitable base comprising cocoa butter or a salicylate. Formulations suitable for vaginal administration may be presented as pessaries, tampons, creams, gells, pastes, foams or spray formulas. Formulations suitable for parenteral administration include aqueous and non-aqueous, isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example ampules and vials, and may be stored in a lyophilized condition requiring only the addition of the sterile liquid carrier, for example water for injections, immediately prior to use. Extemporaneous injection solutions and suspensions may be prepared from sterile powder, granules and tablets of the kind previously described.

<u>Example 1</u>

Dimethylamine was bubbled into 200 ml of THF at -20 °C until 58 g of the gas was dissolved. 7-chloro-1,3-dihydro-5-(1H-pyrrol-2-yl)-1,4-benzodiazepin-2-one (20 g) was then added to the above prepared solution. To this mixture was added a solution of 13 ml of $TiCl_4$ in 50 ml of toluene with constant stirring. Alter stirring at room temperature, 25 ml of brine and 500 ml of ethyl acetate were added. The mixture was then filtered and the filtrate was evaporated to dryness. The residue solid was purified by column chromatography yielding 7.7 g of 7-chloro-N,N-dimethyl-5-(1H-pyrrol-2-yl)-3H-1,4-benzodiazepin-2-amine, m.p. 152-153 °C (acetonitrile).

<u>Example 2</u>

To a mixture of 7-chloro-1,3-dihydro-5-(1H-pyrrol-2-yl)-1,4-benzodiazepin-2-one (20 g), pyrrolidine (52 ml) and THF (800 ml) was added a solution of $TiCl_4$ (12 ml) in toluene (80 ml). After stirring at room temperature, 20 ml water were added to the reaction. Alter 20 minutes of stirring, the mixture was filtered and the filtrate was evaporated to dryness. The residue was purified by chromatography to yield 19.4 g of 7-chloro-5-(1H-pyrrol-2-yl)-2-(1-pyrrolidinyl)-3H-1,4-benzodiazepine. Crystallization from $CH_2Cl_2$/petroleum ether gave 12 g of product, mp 126-128 °C dec.

Example 3

In a manner analogous to example 2, 7-chloro-1,3-dihydro-5-(1H-pyrrol-2-yl)-1,4-benzodiazepin-2-one (0.5 g) was reacted with cyclopropylamine (1.2 ml) and $TiCl_4$ (0.4 ml) in THF (25 ml), resulting in 7-chloro-N-cyclopropyl-5-(1H-pyrrol-2-yl)-3H-1,4-benzodiazepin-2-amine, mp 197-198°C dec.

Example 4

In a manner analogous to example 2, 7-chloro-1,3-dihydro- 5-(1H-pyrrol-2-yl)-1,4-benzodiazepin-2-one (0.5 g) was reacted with diethylamine (1.1 g, 15 mmol) and $TiCl_4$ (0.29 ml) in THF (25 ml), resulting in 7-chloro-N,N-diethyl-5-(1H-pyrrol-2-yl)-3H-1,4-benzodiazepin-2-amine, mp 157°C.

Example 5

In a manner analogous to example 2, 7-chloro-1,3-dihydro- 5-(1H-pyrrol-2-yl)-1,4-benzodiazepin-2-one (0.8 g) was reacted with benzylamine (2.6g) and $TiCl_4$ (0.65 ml) in THF (27 ml), resulting in 7-chloro-N-(phenylmethyl)-5-(1H-pyrrol-2-yl)-3H-1,4-benzodiazepin-2-amine, mp 183-184°C dec.

Example 6

In a manner analogous to example 2, 7-chloro-1,3-dihydro-5-(1H-pyrrol-2-yl)-1,4-benzodiazepin-2-one (0.5 g) was reacted with 2,2-dimethoxyethylamine (1.8 g) and $TiCl_4$ (0.12 ml) in THF (20 ml), resulting in 7-chloro-N-(2,2-dimethoxyethyl)-5-(1H-pyrrol-2-yl)-3H-1,4-benzodiazepin-2-amine, mp 129-130°C.

Example 7

In a manner analogous to example 2, 7-chloro-1,3-dihydro-5-(1H-pyrrol-2-yl)-1,4-benzodiazepin-2-one (0.5 g) was reacted with aniline (1.4g) and $TiCl_4$ (0.28 ml) in THF (20 ml), resulting in 7-chloro-N-phenyl-5-(1H-pyrrol-2-yl)-3H-1,4-benzodiazepin-2-amine, mp 218-220°C.

Example 8

10g of 7-chloro-1,3-dihydro-5-(1H-pyrrol-2-yl)-1,4-benzodiazepin-2-one were dissolved in THF (400 ml) in a 1 litre flask. The flask was placed in the water bath of an ultrasonic apparatus. To the stirred reaction solution was added $P_4S_{10}$ (10 g) and the reaction mixture was irradiated with ultrasound. The temperature was maintained at about 40°C throughout the reaction. Alter 2.5 hours, an additional portion of $P_4S_{10}$ (10g) was added to the mixture and the reaction was continued for an additional 2 hours. The mixture was cooled to ambient temperature and filtered. After washing the solid by-product with $CH_2Cl_2$, the filtrates were combined and approximately 80% of the solvent was removed in vacuo. The residual solution was basified with saturated sodium bicarbonate solution until a pH of 8 was reached. The precipitate was filtered. The filtrate was extracted with $CH_2Cl_2$, followed by concentration in vacuo. The solids were recrystallized in THF/petroleum ether to give 9.9 g of 7-chloro-1,3-dihydro-5-(1H-pyrrol-2yl)-1,4- benzodiazepin-2-thione, m.p. 258-260°C dec.

Example 9

To a solution of 7-chloro-1,3-dihydro-5-(1H-pyrrol-2-yl)-1,4-benzodiazepin-2-thione (0.6 g) in THF (25 ml) was added ethylenediamine (1.8 g). Alter stirring at room temperature, the solvent was removed in vacuo and the residue was extracted with ethylacetate and washed with saturated sodium bicarbonate and brine. Alter drying and evaporation, the product was crystallized from $CH_2Cl_2/CH_3OH$ resulting in 118 mg of N-[7-chloro-5-(1H-pyrrol-2-yl)-3H-1,4- benzodiazepin-2-yl]-1,2-ethanediamine, mp 149-151°C dec.

Example 10

In a manner analogous to example 9, 7-chloro-1,3-dihydro-5-(1H-pyrrol-2-yl)-1,4-benzodiazepin-2-thione (0.55 g) was reacted with ethanolamine (0.61 g) in THF (25 ml) resulting in 7-chloro-N-(2-hydroxyethyl)-5-(1H-pyrrol-2-yl)-3H-1,4-benzodiazepin-2-amine, 68% yield, mp 197-199°C.

Example 11

In a manner analogous to example 9, 7-chloro-1,3-dihydro-5-(1H-pyrrol-2-yl)-1,4-benzodiazepin-2-thione (0.55 g) was reacted with 3-pyrrolidinol (0.17g) in THF (30 ml) resulting in rac-1-[7-chloro-5-(1H-pyrrol-2-yl)-3H-1,4-benzodiazepin-2-yl]-3-pyrrolidinol, 52% yield, mp 232-234 ° C.

Example 12

In a manner analogous to example 9, 7-chloro-1,3-dihydro-5-(1H-pyrrol-2-yl)-1,4-benzodiazepin-2-thione (0.5 g) was reacted with allylamine (2 ml) in THF (25 ml) resulting in 7-chloro-N-(2-propenyl)-5-(1H-pyrrol-2-yl)-3H-1,4-benzodiazepin-2-amine, 22% yield, mp 177-178 ° C.

Example 13

In a manner analogous to example 9, 7-chloro-1,3-dihydro-5-(1H-pyrrol-2-yl)-1,4-benzodiazepin-2-thione (0.56 g) was reacted with propargylamine (1.1 g) in THF (20 ml), resulting in 7-chloro-N-(2-propynyl)-5-(1H-pyrrol-2-yl)-3H-1,4-benzodiazepin-2-amine, 22% yield, mp 171-173 ° C.

Example 14

In a manner analogous to example 9, 7-chloro-1,3-dihydro-5-(1H-pyrrol-2-yl)-1,4-benzodiazepin-2-thione (0.5 g) was reacted with piperazine (1.7 g) in THF (25 ml), resulting in 7-chloro-2-(1-piperazinyl)-5-(1H-pyrrol-2-yl)-3H-1,4-benzodiazepine, 46% yield), mp 171-172 ° C.

Example 15

A solution of 7-chloro-1,3-dihydro-5-(1H-pyrrol-2-yl)-1,4-benzodiazepin-2-one (0.50 g) in 20 ml THF was cooled in an ice bath under argon. Potassium tertbutoxide (0.24 g) was added and the solution was stirred. To this was added diethoxy phosphoryl chloride (0.56 ml) and the solution was stirred at 0 ° C. Piperidine (0.38 ml) was then added and the reaction was allowed to warm up to room temperature. The solvent was then evaporated. The resulting mixture was dissolved in $CH_2Cl_2$, acidified with acetic acid, washed with saturated sodium bicarbonate solution then brine, and dried. Alter evaporation, the solid was recrystallized with methanol and then with $CH_2Cl_2$/petroleum ether to give 7-chloro-2-(1-piperidinyl)-5-(1H-pyrrol-2-yl)-3H-1,4-benzodiazepine, 20% yield, mp 125-126 ° C.

Example 16

A solution of 0.5 g of 7-chloro-1,3-dihydro-5-(1H-pyrrol-2-yl)-1,4-benzodiazepin-2-one in 15 ml of THF was cooled in an ice bath and 0.26 g of potassium tertiary butoxide was added with stirring under nitrogen. Then, 0.46 ml of diethyl chlorophosphate and, after 20 min, 0.5 mlof hydrazine hydrate were added. After stirring at room temperature, the reaction was acidified with acetic acid and partitioned between ethyl acetate and saturated $NaHCO_3$ solution. The organic layer was dried and evaporated. The solid was dissolved in THF/$CH_2Cl_2$ and filtered through silica gel. Elution with ethyl acetate gave a small amount of starting material and methanol removed the product. Crystallization from $CH_2Cl_2$/$CH_3OH$ gave 0.25 g of 7-chloro-2-hydrazino-5-(1H-pyrrol-2-yl)-3H-1,4-benzodiazepine, mp 283-285 ° C.

Example 17

7-chloro-1,3-dihydro-5-(1H-pyrrol-2-yl)-1,4-benzodiazepin-2-thione (0.5 g) was dissolved in anhydrous THF (20 ml). Potassium tertbutoxide (0.224 g) was added and the mixture was stirred. The reaction was then cooled to 0 ° C and methyl iodide (0.15 ml) was added. The mixture was allowed to warm up to room temperature. Alter 2 hours, the solvent was removed in vacuo and the resulting solid was dissolved in $CH_2Cl_2$. The organic phase was washed with brine and dried. Upon removal of the solvent in vacuo and recrystallization in $CH_2Cl_2$/petroleum ether, 0.26 g of 7-chloro-2-(methylthio)-5-(1H- pyrrol-2-yl)-3H-1,4-benzodiazepine was obtained, mp: 163-164 ° C.

Example 18

7-Chloro-2-(methylthio)-5-(1H-pyrrol-2-yl)-3H-1,4-benzodiazepine was dissolved in methanol and 1.1 eq of sodium methoxide was added. The reaction was stirred under argon at room temperature. Then, the solvent was evaporated. The solid residue was dissolved in $CH_2Cl_2$ and the organic layer was washed with brine and dried. Recrystallization in THF/petroleum ether gave 75% yield of 7-chloro-2-methoxy-5-(1H-pyrrol- 2-yl)-3H-1,4-benzodiazepine, mp 156-157 ° C.

The following galenical compositions containing a compound I or a salt thereof as active ingredients as defined above, can be prepared in a manner known per se:

a) Oral liquid formulation:

| Ingredients | mg/formulation |
|---|---|
| Active ingredient | 20.0 mg |
| Methylparaben | 20.0 mg |
| Sucrose | q.s. |
| Flavoring agent | q.s. |
| Citrate buffer | q.s. |
| Purified water q.s. | 5.0 ml |

b) Tablet formulation:

| Ingredients | mg/tablet |
|---|---|
| Active ingredient | 20 mg |
| Starch | 40 mg |
| Avicel | 80 mg |
| Lactose | 274 mg |
| Magnesium stearate | 2 mg |
| | 416 mg |

c) Soil gelatine capsule formulation:

| Ingredients | mg/capsule |
|---|---|
| Active ingredient | 20 mg |
| Ethoxylated Fatty acids | 500 mg |
| PEG 4000 | 100 mg |
| Vegetable oils q.s. to | 1.0 ml |

## Claims

**1.** Benzodiazepines of formula

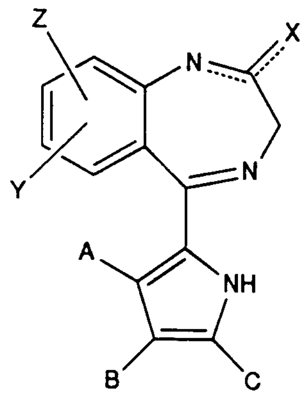

I

wherein

| | |
|---|---|
| X | is N(R,$R^1$), $OR^4$ or $SR^5$, |
| R and $R^1$ | are N($R^2$,$R^3$) or substituted lower alkyl or an optionally substituted $C_3$-$C_7$-cycloalkyl, aryl, aralkyl or $C_2$-$C_6$-aliphatic hydrocarbon group, this aliphatic group having one or more double or triple bond, or one of R and $R^1$ is hydrogen, or |
| N(R,$R^1$) | is an optionally substitued heterocycle with one or more heteroatoms, |
| $R^2$ and $R^3$ | are H, lower alkyl, aryl or aralkyl, |
| $R^4$ | is lower alkyl or has one of the meanings of R or $R^1$ with the exception of H and of N-($R^2$,$R^3$), |
| $R^5$ | is H or lower alkyl or has one of the meanings of R or $R^1$ with the exception of N-($R^2$,$R^3$), |
| Y and Z | are H, $NO_2$, N($R^2$,$R^3$), halogen, $CF_3$, OH, CN or lower alkyl or alkoxy, and |
| A, B and C | are H, $CH_3$, $C_2H_5$, F or Cl, and pharmaceutically acceptable salts thereof. |

**2.** Compounds as in claim 1,wherein X is $SCH_3$, SH, $OCH_3$, $NHNH_2$, NH(cyclopropyl), $NHCH_2CH(OCH_3)_2$, $NHCH_2CH_2OH$, $NHCH_2CH_2NH_2$,$NHCH_2C_6H_5$, $N(CH_3)_2$, $N(CH_2CH_3)_2$, $NHCH_2CH=CH_2$, $NHCH_2C{\equiv}CH$, $NHC_6H_5$, 2-hydroxymethylpyrrolidin-1-yl, 1-pyrrolidinyl, 3-hydroxypyrrolidin-1-yl, 1-piperidinyl, 1-piperazinyl or 4-morpholinyl, Y is Cl, $NO_2$, $NH_2$ or H, and Z, A, B and C are H.

**3.** Compounds as in claim 1, wherein Y is 7-chloro; A, B, C and Z are H and X is $SCH_3$, SH, $OCH_3$, $NHNH_2$, NH(cyclopropyl), $NHCH_2CH(OCH_3)_2$, $NHCH_2CH_2OH$, $NHCH_2CH_2NH_2$, $NHCH_2C_6H_5$, $N(CH_3)_2$, $N(CH_2CH_3)_2$, $NHCH_2CH=CH_2$, $NHCH_2C{\equiv}CH$, $NHC_6H_5$, 1-pyrrolidinyl, 3-hydroxypyrrolidin-1-yl, 1-piperidinyl or 1-piperazinyl.

**4.** A benzodiazepine as in claim 3 of the group of the following:
7-chloro-N,N-dimethyl-5-(1H)-pyrrol-2-yl)-3H-1,4-benzodiazepin-2-amine,
7-chloro-5-(1H-pyrrol-2-yl)-2.(1-pyrrolidinyl)-3H-1,4-benzodiazepine,
7-chloro-N-cyclopropyl-5-(1H-pyrrol-2-yl)-3H-1,4-benzodiazepin-2-amine,
7-chloro-N-(2,2-dimethoxyethyl)-5-(1H-pyrrol-2-yl)-3H-1,4-benzodiazepin-2-amine,
7-chloro-N-(2-hydroxyethyl)-5-(1H-pyrrol-2-yl)-3H-1,4-benzodiazepin-2-amine,
rac-1-[7-chloro-5-(1H-pyrrol-2-yl)-3H-1,4-benzodiazepin-2-yl]-3-pyrrolidinol,
7-chloro-N-(2-propenyl)-5-(1H-pyrrol-2-yl)-3H-1,4-benzodiazepin-2-amine,
7-chloro-N-(2-propynyl)-5-(1H-pyrrol-2-yl)-3H-1,4-benzodiazepin-2-amine,
7-chloro-2-(1-piperidinyl)-5-(1H-pyrrol-2-yl)-3H-1,4-benzodiazepine,
7-chloro-2-hydrazino-5-(1H-pyrrol-2-yl)-3H-1,4-benzodiazepine,
7-chloro-2-methoxy-5-(1H-pyrrol-2-yl)-3H-1,4-benzodiazepine.

**5.** A compound according to any one of claims 1-4 use as a therapeutically active agent, especially for the treatment or prophylaxis of viral infections, particularly of retroviral infections, such as HIV 1 and/or HIV 2 infections, or for protecting cells against such infections.

**6.** A process for preparing a compound as in claim 1, which comprises
a) reacting an amide of formula II or a phosphorylated derivative thereof of formula III

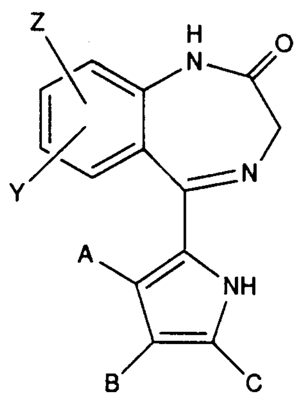 II 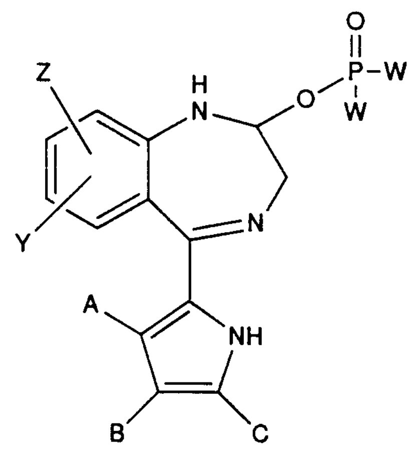 III

wherein W is lower alkoxy or an amine functional group, with an amine of formula HN(R,$R^1$), or

b) reacting an amide II with a thiation reagent and,

c) if desired, S-alkylating a compound of formula I, wherein X is S with a compound of formula $R^5$-L wherein L is a leaving group, and

d) if desired, reacting a compound of formula I, wherein X is S-$R^5$ with an alkali metal alkoxide, and

e) if desired, reacting a compound of formula I, wherein X is S, $SR^5$ or $OR^4$ with an amine HN(R,$R^1$), and

f) if desired, adding a substituent in the benzene moiety of a compound of formula I or interverting functional substituents in said moiety.

**7.** A medicament, especially for the treatment or prophylaxis of viral infections, particularly of retroviral infections, such as HIV 1 and/or HIV 2 infections, or for protecting cells against such infections, containing as active pharmaceutical ingredient a compound as in claim 1, 2 or 3 and, optionally, a second antiviral agent, especially a reverse transcriptase inhibitor, such as ddC, AZT, a HIV-protease inhibitor, $\alpha$-, $\beta$- and/or $\gamma$-interferon, interleukin-2 and/or GM-CSF.

**8.** The use of a compound as in claim 1,2 or 3, for the manufacture of a medicament especially for the treatment or prophylaxis of viral infections, particularly of retroviral infections, such as HIV 1 and/or HIV 2 infections, or for protecting cells against such infections.

European Patent Office

# EUROPEAN SEARCH REPORT

Application Number

EP 91114730.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE - A1 - 2 017 060 (SUMITOMO) * Claim 1; page 33, line 7 * -- | 1 | C 07 D 403/04<br>A 61 K 31/55 |
| A | DE - A1 - 2 223 648 (HOFFMANN) * Claims 1,25 * -- | 1,7 | |
| A | CHEMICAL ABSTRACTS, vol. 113, No. 7, August 13, 1990, Columbus, Ohio, USA KURIHARA, TAKUSHI et al. "Synthesis of 5-(4-pyrazolyl and 4-isoxazolyl)-1,3-dihydro-2H-1,4-bezodiazepin-2-ones" page 699, column 1, abstract-No. 59 122w & Heterocycles 1989, 29(10), 2007-21 -- | 1 | |
| | | | TECHNICAL FIELDS SEARCHED (Int. Cl.5) |
| A | CHEMICAL ABSTRACTS, vol. 112, No. 19, May 7, 1990, Columbus, Ohio, USA PAUWELS, RUDI et al. "Potent and selective inhibition of HIV-1 replication in vitro by a novel series of TIBO derivatives" page 418, column 2, abstract -No. 175 463m & Nature (London) 1990, 343 (6257), 470-4 -- | 7,8 | C 07 D 403/00 |
| P,A | EP - A1 - 0 393 530 (BOEHRINGER) * Claim 1 * -- | 7,8 | |
| P,A | EP - A2 - 0 393 604 (BOEHRINGER) | 7,8 | |

The present search report has been drawn up for all claims

| Place of search | Date of completion of the search | Examiner |
|---|---|---|
| VIENNA | 04-11-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-
EP 91114730.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | * Claim 10 * <br> -- | | |
| P,A | EP - A1 - 0 417 840 (JANSSEN) <br> * Claim 9; formulas XXXI, XXXII; page 19, line 22 - page 20, line 34 * <br> ---- | 1,7 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 04-11-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)